# EUROPEAN PATENT SPECIFICATION

(11) **EP 0 693 285 B1**
(45) Date of publication and mention of the grant of the patent: **06.02.2002**
(21) Application number: 95305083.8
(22) Date of filing: 20.07.1995
(51) Int. Cl.: A61K 31/66, A61K 31/6615, A61K 31/675, A61K 31/381

(54) **Pharmaceutical compositions containing a bisphosphonate and an anti-resorptive agent for inhibiting bone loss**
Bisphosphonat und anti-Resorption Agentien, enthaltende Zusammensetzungen zur Hemmung der Knochenschwund
Composition pharmaceutique contenant un bisphosphonate et un agent anti-resorptif pour inhiber la perte osseuse

(30) Priority: 22.07.1994 US 279363
(43) Date of publication of application: 24.01.1996
(73) Proprietor: ELI LILLY AND COMPANY, Indianapolis, Indiana 46285 (US)
(72) Inventor: Black, Larry John, Franklin, Indiana 46131 (US); Cullinan, George Joseph, Trafalgar, Indiana 46181 (US)
(74) Representative: Vaughan, Jennifer Ann

(56) References cited:
- J. KOCI N: "Advances in the pharmacotherapy of osteoporosis." VNITRINI LEKARSTVI, vol. 41, no. 3, 1995, pages 207-209, XP002057991

## Description

Current major diseases or conditions of bone which are of public concern include post-menopausal osteoporosis, senile osteoporosis, patients undergoing long-term treatment of corticosteroids, side effects from glucocorticoid or steroid treatment, patients suffering from Cushings's syndrome, gonadal dysgensis, periarticular erosions in rheumatoid arthritis, osteoarthritis, Paget's disease, osteohalisteresis, osteomalacia, hypercalcemia of malignancy, osteopenia due to bone metastases, periodontal disease, and hyperparathyroidism. All of these conditions are characterized by bone loss, resulting from an imbalance between the degradation of bone (bone resorption) and the formation of new healthy bone. This turnover of bone continues normally throughout life and is the mechanism by which bone regenerates. However, the conditions stated above will tip the balance towards bone loss such that the amount of bone resorbed is inadequately replaced with new bone, resulting in net bone loss.

One of the most common bone disorders is post-menopausal osteoporosis which affects an estimated 20 to 25 million women in the United States alone. Women after menopause experience an increase in the rate of bone turnover with resulting net loss of bone, as circulating estrogen levels decrease. The rate of bone turnover differs between bones and is highest in sites enriched with trabecular bone, such as the vertebrae and the femoral head. The potential for bone loss at these sites immediately following menopause is 4-5% per year. The resulting decrease in bone mass and enlargement of bone spaces leads to increased fracture risk, as the mechanical integrity of bone deteriorates rapidly.

At present, there are 20 million people with detectable vertebral fractures due to osteoporosis and 250,000 hip fractures per year attributable to osteoporosis in the U.S. The latter case is associated with a 12% mortality rate within the first two years and 30% of the patients will require nursing home care after the fracture. Therefore, bone disorders are characterized by a noticeable mortality rate, a considerable decrease in the survivor's quality of life, and a significant financial burden to families.

Essentially all of the conditions listed above would benefit from treatment with agents which inhibit bone resorption. Bone resorption proceeds by the activity of specialized cells called osteoclasts. Osteoclasts are unique in their ability to resorb both the hydroxyapatite mineral and organic matrix of bone. They are somewhat similar to the cartilage resorbing cells, termed chondroclasts. It is for this reason that potent inhibitors of osteoclastic bone resorption may also inhibit the cell-mediated degradation of cartilage observed in rheumatoid arthritis and osteoarthritis.

Therapeutic treatments to impede net bone loss include the use of estrogens. Estrogens have been shown clearly to arrest the bone loss observed after menopause and limit the progression of osteoporosis; but patient compliance has been poor because of estrogen side-effects. These side effects include resumption of menses, mastodynia, increase in the risk of uterine cancer, and possibly an increase in the risk of breast cancer.

Alternatively, calcitonin has been used to treat osteoporotic patients. Salmon calcitonin has been shown to directly inhibit the resorption activity of mammalian osteoclasts and is widely prescribed in Italy and Japan. However, calcitonins are prohibitively expensive to many and appear to be short-lived in efficacy. That is, osteoclasts are able to "escape" calcitonin inhibition of resorption by down-regulating calcitonin receptors. Therefore, recent clinical data suggest that chronic treatment with calcitonin may not have long term effectiveness in arresting the post-menopausal loss of bone. There continues to be great interest in research directed to novel therapies to inhibit bone loss.

This invention provides the use of a first compound which is a 2-phenyl-3-aroylbenzo[b]thiophene having the formula 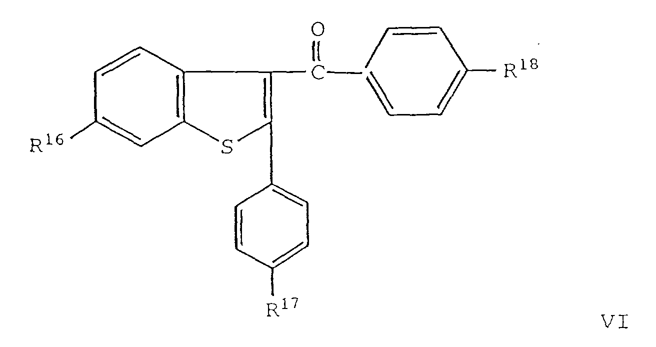 where
R¹⁶ is hydrogen, hydroxy or C₁-C₅ alkoxy C₁-C₇ alkanoyloxy, C₃-C₇ cycloalkanoyloxy, (C₁-C₆ alkoxy)-C₁-C₇ alkanoyloxy, substituted or unsubstituted aroyloxy, or substituted or unsubstituted aryloxycarbonyloxy;
R¹⁷ is hydrogen, hydroxy, C₁-C₅ alkoxy, adamantoyloxy, chloro, bromo, C₁-C₇ alkanoyloxy, C₃-C₇ cycloalkanoyloxy, (C₁-C₆ alkoxy)-C₁-C₇ alkanoyloxy, substituted or unsubstituted aroyloxy, or substituted or unsubstituted aryloxycarbonyloxy;
R¹⁸ is -O-CH₂-CH₂-X'-NR¹⁹R²⁰;
X' is a bond or -CH₂-, R¹⁹ and R²⁰ are independently C₁-C₄ alkyl or are taken together with the nitrogen atom to which they are bonded to constitute a pyrrolidinyl, piperidinyl, hexamethyleneiminyl, or morpholinyl ring; and pharmaceutically acceptable acid addition salts and solvates thereof in the preparation of a medicament in a synergistic combination with a second compound being a bisphosphonate, and pharmaceutically acceptable salts and solvates thereof, for inhibiting bone loss in a human.

Also encompassed by the invention are combination pharmaceutical formulations and salts.

This invention concerns the discovery that combination therapy for humans, comprising administering a component from the first group of compounds, as defined above, with a synergistic amount of bisphosphonate, is useful in the inhibition of bone loss. The therapy may be sequential, concurrent or simultaneous, with the latter two being preferred.

The general chemical terms used in the description of the compounds of this invention have their usual meanings. For example, the term "alkyl" by itself or as part of another substituent means a straight or branched chain alkyl radical having the stated number of carbon atoms such as methyl, ethyl, propyl, and isopropyl and higher homologues and isomers where indicated.

The term "alkoxy" means an alkyl group having the stated number of carbon atoms linked to the parent moiety by an oxygen atom, such as methoxy, ethoxy, propoxy, butoxy, pentyloxy, and hexyloxy and also includes branched chain structures such as, for example, isopropoxy and isobutoxy.

The term "substituted alkyl" includes an alkyl substituted once or more with substitutents known in the art. As this term is used in conjunction with the bisphosphonates, those references in this art would disclose such substitutents.

The term "C₁-C₇-alkanoyloxy" means a group -O-C(O)-R^{a} where Ra is hydrogen or C₁-C₆ alkyl and includes formyloxy, acetoxy, propanoyloxy, butanoyloxy, pentanoyloxy, hexanoyloxy, and the like and also includes branched chain isomers such as, for example, 2,2-dimethylpropanoyloxy, and 3,3-dimethylbutanoyloxy.

Analogously, the term C₄-C₇ cycloalkanoyloxy" means a group -O-C(O)-(C₃-C₆ cycloalkyl) where the C₃-C₆ alkyl group includes cyclopropyl, cyclobutyl, cyclopentyl and cyclohexyl.

The term "(C₁-C₆-alkoxy)-C₁-C₇-alkanoyloxy" means a group -O-C(O)-R^{b}-O-(C₁-C₆ alkyl) where R^{b} is a bond (C₁-C₆ alkoxycarbonyloxy) or C₁-C₆ alkanediyl and includes, for example, methoxycarbonyloxy, ethoxycarbonyloxy, propoxycarbonyloxy, butoxycarbonyloxy, methoxyacetoxy, methoxypropanoyloxy, methoxybutanoyloxy, methoxy-pentanoyloxy, methoxyhexanoyloxy, ethoxyacetoxy, ethoxypropanoyloxy, ethoxybutanoyloxy, ethoxypentanoyloxy, ethoxyhexanoyloxy, propoxyacetoxy, propoxypropanoyloxy, propoxybutanoyloxy, and the like.

The term "unsubstituted or substituted aroyloxy" means a group -O-C(O)-aryl where aryl is a phenyl, naphthyl, thienyl or furyl group that is, as to each group, unsubstituted or monosubstituted with a hydroxyl, halo, C₁-C₃ alkyl, or C₁-C₃ alkoxy.

The term "unsubstituted or substituted aryloxycarbonyloxy" means a group -O-C(O)-O-aryl where aryl is a phenyl, naphthyl, thienyl or furyl group that is, as to each group, unsubstituted or monosubstituted with a hydroxyl, halo, C₁-C₃ alkyl or C₁-C₃ alkoxy.

The term "halo" means chloro, fluoro, bromo or iodo.

The term "inhibit" is defined to include its generally accepted meaning which includes preventing, prohibiting, restraining, and slowing, stopping or reversing progression, or severity, and holding in check and/or treating existing characteristics. The present use of synergistic combinations for the manufacture of a medicament for inhibiting bone loss includes both medical therapeutic and/or prophylactic treatment, as appropriate.

The term "pharmaceutically acceptable salts" refers to salts of the compounds of the above classes which are substantially non-toxic to living organisms. Typical pharmaceutically acceptable salts include those salts prepared by reaction of a compound of the above class with a pharmaceutically acceptable mineral or organic acid, or a pharmaceutically acceptable alkali metal or organic base, depending on the types of substituents present on the compound.

Examples of pharmaceutically acceptable mineral acids which may be used to prepare pharmaceutically acceptable salts include hydrochloric acid, phosphoric acid, sulfuric acid, hydrobromic acid, hydroiodic acid, phosphorous acid and the like. Examples of pharmaceutically acceptable organic acids which may be used to prepare pharmaceutically acceptable salts include aliphatic mono and dicarboxylic acids, oxalic acid, carbonic acid, citric acid, succinic acid, phenyl-substituted alkanoic acids, aliphatic and aromatic sulfonic acids and the like. Such pharmaceutically acceptable salts prepared from mineral or organic acids thus include hydrochloride, hydrobromide, nitrate, sulfate, pyrosulfate, bisulfate, sulfite, bisulfite, phosphate, monohydrogenphosphate, dihydrogenphosphate, metaphosphate, pyrophosphate, hydroiodide, hydrofluoride, acetate, propionate, formate, oxalate, citrate, lactate, p-toluenesulfonate, methanesulfonate, maleate, and the like.

Many compounds of the above classes which contain a carboxy, carbonyl, or hydroxy or sulfoxide group may be converted to a pharmaceutically acceptable salt by reaction with a pharmaceutically acceptable alkali metal or organic base. Examples of pharmaceutically acceptable organic bases which may be used to prepare pharmaceutically acceptable salts include ammonia, amines such as triethanolamine, triethylamine, ethylamine, and the like. Examples of pharmaceutically acceptable alkali metal bases included compounds of the general formula MOZ, where M represents an alkali metal atom, e.g. sodium, potassium, or lithium, and Z represents hydrogen or C₁-C₄ alkyl.

It should be recognized that the particular anion or cation forming a part of any salt of this invention is not critical, so long as the salt, as a whole, is pharmacologically acceptable and as long as the anion or cationic moiety does not contribute undesired qualities.

In addition, some of the compounds disclosed as useful in the preparation of medicaments of the present invention may form solvates with water or common organic solvents. Such solvates are included within the scope of the present invention and solvates thereof.

The class of compounds known as bisphosphonates includes those compounds which contains a di-phosphonic acid moiety separated by a carbon link and include a variety of side-chains, usually containing a basic function. The compounds have the following general structure: 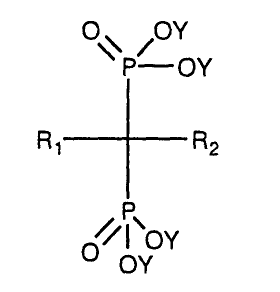 Y, R₁ and R₂ may be those substitutents as defined in US Patent 5,139,786, and EPO Publication 0416689A2, published March 13, 1991, although not limited to such.

Pharmacologically, these compounds have been shown to slow or stop bone resorption by inhibiting osteoclast cell function. Several compounds of this class are currently undergoing clinical evaluation for the treatment of post-menopausal osteoporosis. Many of these compounds are also being evaluated for the treatment of Paget's Disease and hypercalcemia malignancy and several have been approved.

The art refers to three different generations of bisphosphonates. The first generation usually refers to the compound etidronate. This compound is being marketed for the treatment of Paget's disease and hypercalcemia malignacy.

The second generation of bisphosphonates refers to the compounds clodronate and pamidronate. Clodronate is marketed for Paget's disease and hypercalcemia maligancy. Pamidronate will probably be approved for osteoporosis in some European countries in the near future.

The third generation of bis-phosphonates refer to alendronate, risedronate, and tiludronate and a host of lesser known compounds. Pharmacologically, these compounds are much more potent and are claimed to have fewer side-effects.

The structures of some bisphosphonate compounds are as follows:

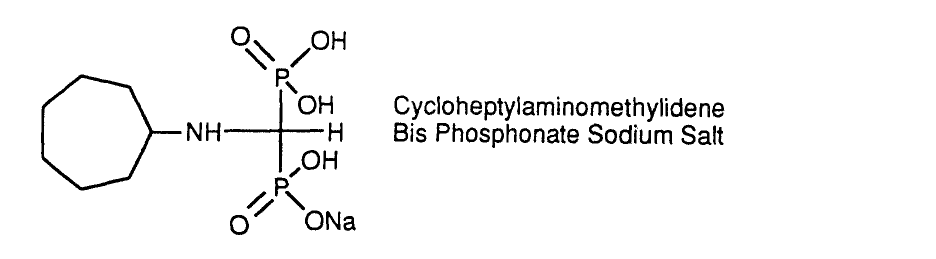

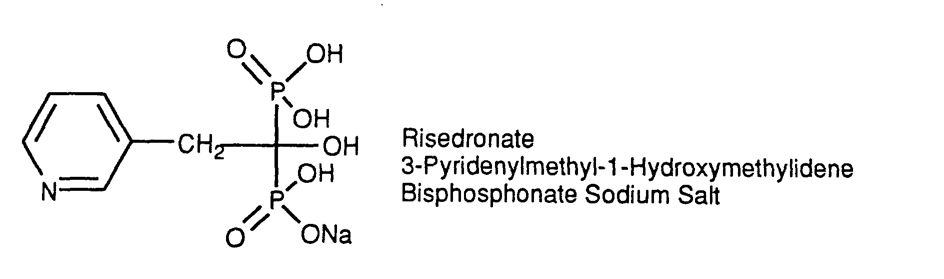

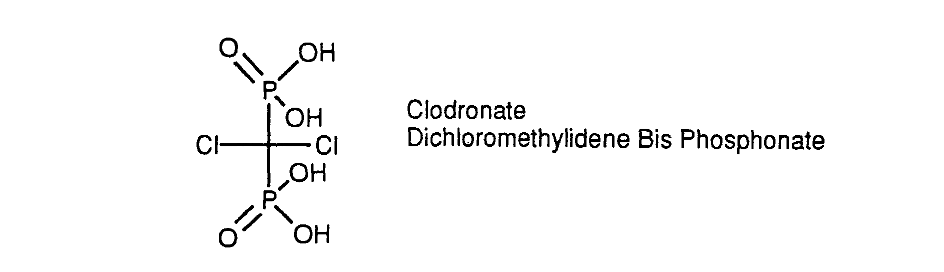

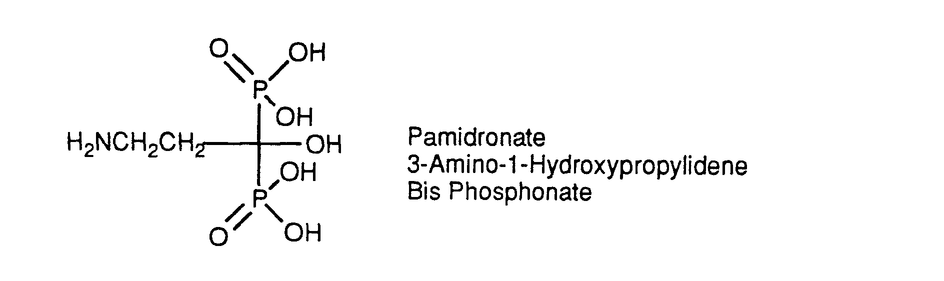

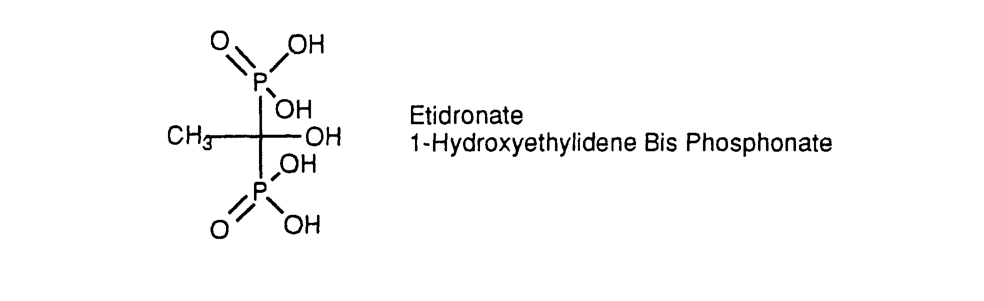

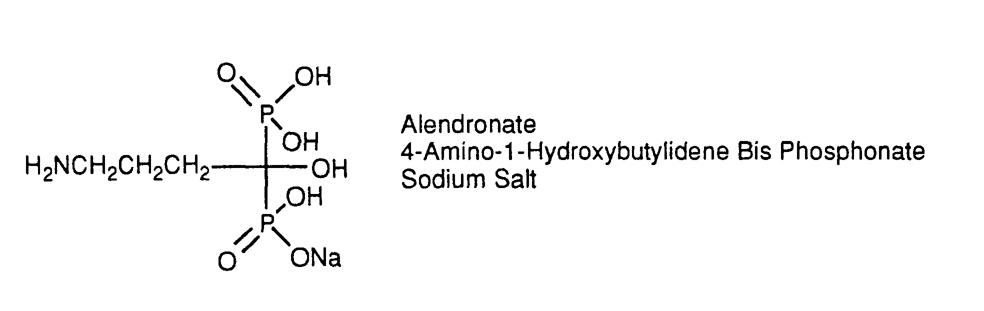

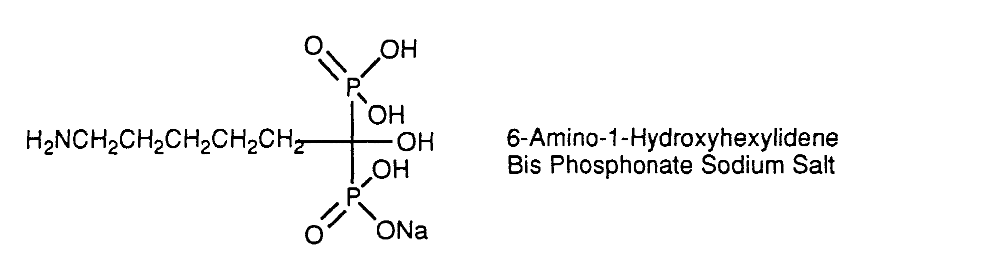

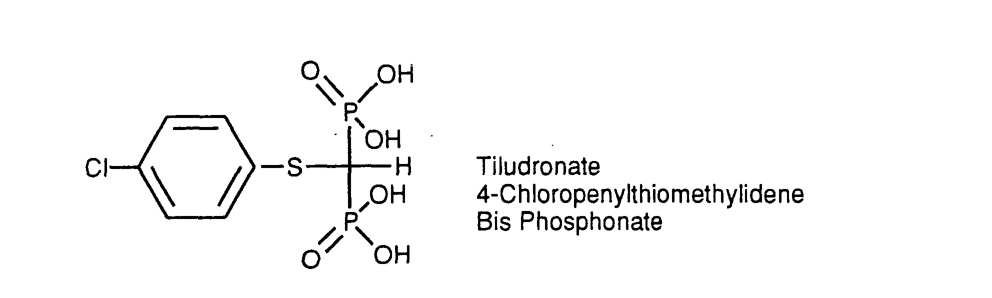

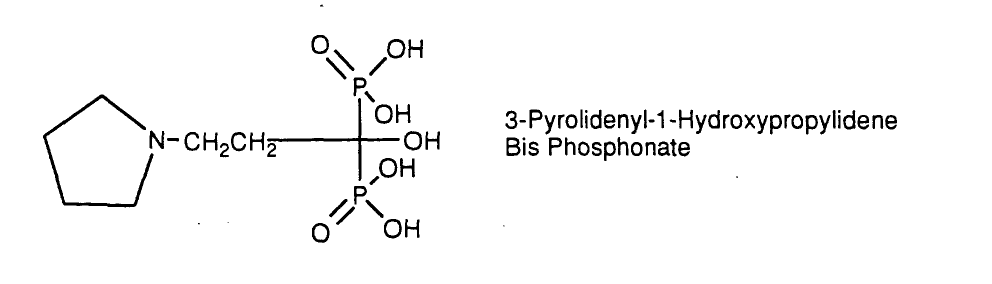

Preferred are alendronate, pamidronate, risedronate, cycloheptylaminomethylidene bis phosphonate, and 3-pyrolidenyl-1-hydroxy-propylidene bisphosphosphonate, and salts and solvates thereof.

Bisphosphonates appear to have the potential of treating osteoporosis; however, they also apppear to have potential detrimental side-effects:
1) they have the potential of inhibiting bone formation as well as resorption;
2) they are poorly adsorbed via oral adminstration and are known to cause G.I. irritation;
3) they have extremely long half-lives in bone;
4) they may all have the potential for causing osteomalacia; and
5) there is concern as to the bio-mechanical strength of the bones treated with bis-phosphonates.

In general, it is felt that these compounds may have great promise for treating osteoporosis; however, there is concern as to their long term effects

Therefore, it is reasonable that the minimal exposure of the osteoporotic patient to these compounds would be desirable. Reducing exposure without sacrificing efficacy might be achievable by either using the bis-phosphonates for a limited period of time (cyclically) or by enhancing their efficacy by the inclusion of another anti-resorptive agent, working by a different mechanism of action.

The 2-Phenyl-3-aroylbenzo[b]thiophene compounds, (Z-triarylpropenones) are disclosed in and prepared according to procedures described in U.S. Patent No. 4,133,814; U.S. Patent No. 4,418,068; and Jones, et al., J. Med. Chem., 27, 1057-1066 (1984).

Specific illustrative compounds within this class include Raloxifene [6-hydroxy-2-(4-hydroxyphenyl)benzo[b]thien-3-yl][4-[2-(1-piperidinyl)ethoxy]phenyl]methanone hydrochloride, formerly keoxifene; and [6-hydroxy-2-(4-hydroxyphenyl)benzo[b]thien-3-yl] [4-[2-(1-pyrrolidinyl)ethoxy]phenyl]methanone Hydrochloride.

The 2-phenyl-3-aroylbenzo[b]thiophenes are exemplified by those in U.S. Patent 4,133,814 and have the formula 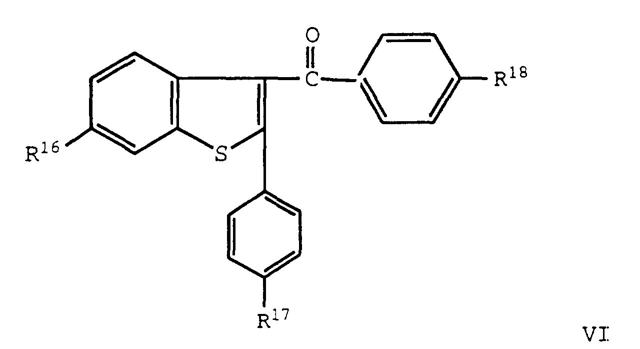 where
R¹⁶ is hydrogen, hydroxy, C₁-C₅ alkoxy, C₁-C₇ alkanoyloxy, C₃-C₇ cycloalkanoyloxy, (C₁-C₆ alkoxy)-C₁-C₇ alkanoyloxy, substituted or unsubstituted aroyloxy, or substituted or unsubstituted aryloxycarbonyloxy;
R¹⁷ is hydrogen, hydroxy, C₁-C₅ alkoxy, adamantoyloxy, chloro, bromo, C₁-C₇ alkanoyloxy, C₃-C₇ cycloalkanoyloxy, (C₁-C₆ alkoxy)-C₁-C₇ alkanoyloxy, substituted or unsubstituted aroyloxy, or substituted or unsubstituted aryloxycarbonyloxy;
R¹⁸ is -O-CH₂-CH₂-X'-NR¹⁹R²⁰; X' is a bond or -CH₂-, R¹⁹ and R²⁰ are independently C₁-C₄ alkyl or are taken together with the nitrogen atom to which they are bonded to constitute a pyrrolidinyl, piperidinyl, hexamethyleneiminyl, or morpholinyl ring; and pharmaceutically acceptable acid addition salts and solvates thereof.

Methods of synthesizing these compounds are disclosed in U.S. Patent 4,133,814. Raloxifene, and its preparation, are described in U.S. Patent 4,418,068.

The preferred compounds useful in the preparation of medicaments of the present invention are benzothiopenes having the formula: 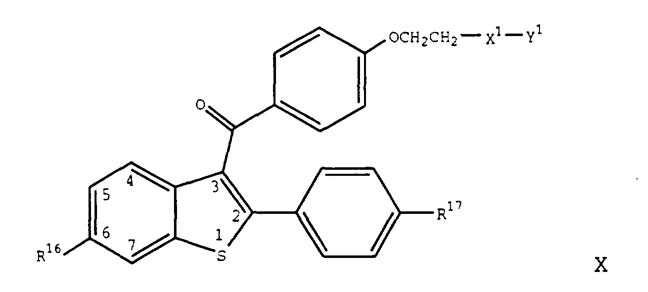 wherein
X¹ is a bond or -CH₂-;
R¹⁶ is hydroxyl, methoxy, C₁-C₇ alkanoyloxy, C₃-C₇ cycloalkanoyloxy, (C₁-C₆ alkoxy)-C₁-C₇ alkanoyloxy, substituted or unsubstituted aroyloxy, or substituted or unsubstituted aryloxycarbonyloxy;
R¹⁷ is hydrogen, hydroxyl, chloro, bromo, methoxy, C₁-C₇ alkanoyloxy, C₃-C₇ cycloalkanoyloxy, (C₁-C₆ alkoxy)-C₁-C₇ alkanoyloxy, substituted or unsubstituted or aroyloxy, or substituted or unsubstituted aryloxycarbonyloxy;
Y¹ is a heterocyclic ring selected from the group consisting of pyrrolidinyl, piperidinyl, or hexamethyleneiminyl; and pharmaceutically acceptable salts and solvates thereof. Particularly preferred are raloxifene and its pyrrolidinyl analog.

Included within the invention are salt formation products, preferably consisting of one molecule of the acidic bis-phosphonate and one molecule of the basic compound of the first group. Preferred salts are raloxifene/alendronate; raloxifene/pamidronate; raloxifene/risedronate; raloxifene/cycloheptyl amino methylidene bisphosphonate, and raloxifene/3-pyrolidenyl-1-hydroxy propylidene bisphosphonate.

The compounds utilized in the preparation of medicaments of the present invention are effective over a wide dosage range. For example, dosages of compounds of the first group per day will normally fall within the range of about 0.01 to about 1000 mg/kg of body weight. In the treatment of adult humans, the range of about 10 to about 600 mg/day, in single or divided doses, is preferred. The amount of bisphonate will fall within 5 mg/day at 400 mg/day. However, it will be understood that the amount of the compounds actually administered will be determined by a physician in light of the relevant circumstances including the condition to be treated, the choice of compounds to be administered, the age, weight, and response of the individual patient, the severity of the patient's symptoms and the chosen route of administration. While the present compounds are preferably administered orally, the compounds may also be administered by a variety of other routes such as the transdermal, subcutaneous, intranasal, intramuscular and intravenous routes.

While it is possible to administer the compounds directly, the compounds are preferably employed in the form of a pharmaceutical formulation comprising a pharmaceutically acceptable carrier, diluent or excipient and a compound of the invention. Such formulations will contain from about 0.01 percent to about 99 percent of the compounds.

In making the formulations of the present invention, the active ingredients will usually be mixed with at least one carrier, or diluted by at least one carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the formulations can be in the form of tablets, granules, pills, powders, lozenges, sachets, cachets, elixirs, emulsions, solutions, syrups, suspensions, aerosols (as a solid or in a liquid medium) and soft and hard gelatin capsules.

Examples of suitable carriers, diluents and excipients include lactose, dextrose, sucrose, sorbitol, mannitol, starches, gum acacia, calcium phosphate, alginates, liquid paraffin, calcium silicate, microcrystalline cellulose, polyvinyl pyrrolidone, cellulose, tragacanth, gelatin, syrup, methyl cellulose, methyl- and propyl-hydroxybenzoates, vegetable oils, such as olive oil, injectable organic esters such as ethyl oleate, talc, magnesium stearate, water and mineral oil. The formulations may also include wetting agents, lubricating, emulsifying and suspending agents, preserving agents, sweetening agents, perfuming agents, stabilizing agents or flavoring agents. The formulations of the invention may be formulated so as to provide quick, sustained or delayed release of the active ingredient after administration to the patient by employing procedures well-known in the art.

For oral administration, the compounds can be admixed with carriers and diluents and molded into tablets or enclosed in gelatin capsules.

The compositions are preferably formulated in a unit dosage form, each dosage containing from about 1 to about 500 mg, more usually about 5 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier, diluent or excipient therefor.

More particularly, there are three different, basic formulations envisioned within this invention.

### 1) Separate, co-administered dosage formulations

This formulation consists of each drug separately formulated for parenteral or oral adminstration in manners well known to those skilled in the art and as particularly taught in references of each of the compounds cited. Because two separate formulations are being co-adminstered, each formulation, especially those by the oral route, would be color-coded or otherwise easily indentifiably labelled to avoid confusion by both patient or physician. Since a concept of this invention is to minimize the exposure of the patient to high doses of the bisphosphonate while maximizing the efficacy, the envisioned protocols for use of this invention would necessiate a short term or cyclic use of the bis-phosphonate and a continous use of a compound of the first group.

### 2) Single Mixture Formulations

One way to avoid possible confusion and which would allow for various strengths of the two different drugs would be to combine the two entities in a simple mixture in forms well known and taught in the art. A patient could an orange tablet containing 50 mg of, for example, raloxifene and 25 mg of risedronate, once a day, for two weeks, followed in continuance of the blue tablet of 50mg of raloxifene.

### 3) Single Molar Defined Salt Formulations

This formulation, where each drug is preferably the counter ion for the other, would lead to a salt of defined chemical composition. This would aid in consistancy and homogeneity of the preparation and may aid in absorption of the bis-phosphonate by oral adminstration.

Since there is a great deal of concern about the side-effects of bis-phosphonate therapy, prolonged, continous use of the bis-phosphonate would not be recommended, rather a cyclic regiment would be more appropriate. An example of such a cyclic protocol is taught in the art in regard to the use of bis-phosphonates in the treatment of Paget's Disease and specifically for the treatment of osteoporosis in "Watts, N.B., *et al.,* The New England J. of Medicine, 323(2), p.73-79.

In order to more fully illustrate the operation of this invention, the following examples of formulations are provided. The examples are illustrative only and are not intended to limit the scope of the invention. The formulations may employ as active ingredients any of the compounds described above.

### FORMULATION 1

Hard gelatin capsules are prepared using the following ingredients:

| | Amt. per Capsule | Concentration by Weight (percent) |
|---|---|---|
| Active Ingredient(s) | 250 mg | 55.0 |
| Starch dried | 220 mg | 43.0 |
| Magnesium stearate | 10 mg | 2.0 |
| | 460 mg | 100.0 |

The above ingredients are mixed and filled into hard gelatin capsules in 460 mg quantities.

### FORMULATION 2

Capsules each containing 20 mg of medicament are made as follows:

| | Amt. per Capsule | Concentration by Weight (percent) |
|---|---|---|
| Active Ingredient(s) | 20 mg | 10.0 |
| Starch | 89 mg | 44.5 |
| Microcrystalline cellulose | 89 mg | 44.5 |
| Magnesium stearate | 2 mg | 1.0 |
| | 200 mg | 100.0 |

The active ingredient(s), cellulose, starch and magnesium stearate are blended, passed through a 0.3 mm No. 45 mesh U.S. sieve and filled into a hard gelatin capsule.

### FORMULATION 3

Capsules each containing 100 mg of active ingredient(s) are made as follows:

| | Amt. per Capsule | Concentration by Weight (percent) |
|---|---|---|
| Active Ingredient(s) | 100 mg | 29.0 |
| Polyoxyethylenesorbitan monooleate | 50 mcg | 0.02 |
| Starch powder | 250 mg | 71.0 |
| | 250.05 mg | 100.02 |

The above ingredients are thoroughly mixed and placed in an empty gelatin capsule.

### FORMULATION 4

Tablets each containing 10 mg of active ingredient(s) are made up as follows:

| | Amt. per Capsule | Concentration by Weight (percent) |
|---|---|---|
| Active Ingredient(s) | 10 mg | 10.0 |
| Starch | 45 mg | 45.0 |
| Microcrystalline cellulose | 35 mg | 35.0 |
| Polyvinyl pyrrolidone (as 10% solution in water) | 4 mg | 4.0 |
| Sodium carboxyethyl starch | 4.5 mg | 4.5 |
| Magnesium stearate | 0.5 mg | 0.5 |
| Talc | 1 mg | 1.0 |
| | 100 mg | 100.0 |

The active ingredient(s), starch and cellulose are passed through a 0.3 mm No. 45 mesh U.S. sieve and mixed thoroughly. The solution of polyvinylpyrrolidone is mixed with the resultant powders which are then passed through a No. 14 mesh U.S. sieve. The granule so produced is dried at 50°-60°C and passed through a No. 18 mesh U.S. sieve. The sodium carboxymethyl starch, magnesium stearate and talc, previously passed through a No. 60 mesh U.S. sieve, are then added to the granule which, after mixing, is compressed on a tablet machine to yield a tablet weighing 100 mg.

### FORMULATION 5

A tablet formula may be prepared using the ingredients below:

| | Amt. per Capsule | Concentration by Weight (percent) |
|---|---|---|
| Active Ingredient(s) | 250 mg | 38.0 |
| Cellulose microcrystalline | 400 mg | 60.0 |
| Silicon dioxide fumed | 10 mg | 1.5 |
| Stearic acid | 5 mg | 0.5 |
| | 665 mg | 100.0 |

The components are blended and compressed to form tablets each weighing 665 mg.

### FORMULATION 6

Suspensions each containing 5 mg of medicaments per 40 ml dose are made as follows:

| | Per 5 ml of suspension |
|---|---|
| Active Ingredient(s) | 5 mg |
| Sodium carboxymethyl cellulose | 50 mg |
| Syrup | 1.25 ml |
| Benzoic acid solution | 0.10 ml |
| Flavor | q.v. |
| Color | q.v. |
| Water | q.s. to 5 ml |

The medicament is passed through a 0.3 mm No. 45 mesh U.S. sieve and mixed with the sodium carboxymethylcellulose and syrup to form a smooth paste. The benzoic acid solution, flavor and color is diluted with some of the water and added, with stirring. Sufficient water is then added to produce the required volume.

### FORMULATION 7

An aerosol solution is prepared containing the following components:

| | Concentration by Weight (%) |
|---|---|
| Active Ingredient(s) | 0.25 |
| Ethanol | 29.75 |
| Propellant 22 (Chlorodifluoromethane) | 70.00 |
| | 100.00 |

The active compound(s) are mixed with ethanol and the mixture added to a portion of the propellant 22, cooled to -30°C and transferred to a filling device. The required amount is then fed to a stainless steel container and diluted further with the remaining amount of propellant. The valve units are then fitted to the container.

The following is an example of the preparation of a salt formula between a bisphosphonate and a compound from group 1.

### Combination Salt of [2-(4-Hydroxyphenyl)-6-hydroxybenzo[B]thien-3-yl] [4-[2-(1-piperidenyl)ethoxy]phenyl]methanone and 4-amino-1-hvdroxybutyl-1,1-bis phosphonate

2.37g of [2-(4-hydroxyphenyl)-6-hydroxybenzo[B]thien-3-yl]4-[2-(1-piperidenyl)ethoxy]phenyl] methanone (0.005 mol) was dissolved in 25 mL of EtOH.

1.36g of 4-amino-1-hydroxybutyl-1, 1-bisphosphonate mono sodium (0.005 mol) was dissolved in 25 mL of water and 5 mL of 1N HCl (0.005) was added. The reaction mixture was evaporated to a gummy white solid and redissolved in 10 mL of water. This aqueous solution was then added to the EtOH solution prepared above. This reaction mixture was heated on a steam bath for one hour in order to dissolve all the components. The reaction mixture was evaporated to a white amorphous powder and dried under vaccuum at room temperature for 24-hours.

This yielded 3.6 g of the title compound as a white amorphous powder.
MS: m/e=780 (M-1)
474 (Raloxifene base +1)
309 (Bis-phosphonate-NaCl +1)
EA: Clac: C, 49.21; h,5.16; N,3.59 Found: C,41.80; H, 5.06; N,3.57.

In the following, a model of post-menopausal osteoporosis was used in which effects of different treatments upon femur density were determined.

Seventy-five day old female Sprague Dawley rats (weight range of 225 to 275 g) were obtained from Charles River Laboratories (Portage, MI). They were housed in groups of 3 and had *ad libitum* access to food (calcium content approximately 1%) and water. Room temperature was maintained at 22.2° ± 1.7° C with a minimum relative humidity of 40%. The photoperiod in the room was 12 hours light and 12 hours dark.

One week after arrival, the rats underwent bilateral ovariectomy under anesthesia (44 mg/kg Ketamine and 5 mg/kg Xylazine (Butler, Indianapolis, IN) administered intramuscularly). Treatment with vehicle or the indicated compound was initiated either on the day of surgery following recovery from anesthesia or 35 days following the surgery.

Oral dosage was by gavage in 0.5 mL of 1% carboxymethylcellulose (CMC).

Body weight was determined at the time of surgery and weekly during the study, and the dosage was adjusted with changes in body weight. Vehicle-treated ovariectomized (ovex) rats and non-ovariectomized (intact) rats were evaluated in parallel with each experimental group to serve as negative and positive controls.

The rats were treated daily for 35 days (6 rats per treatment group) and sacrificed by decapitation on the 36th day. The 35-day time period was sufficient to allow maximal reduction in bone density, measured as described infra. At the time of sacrifice, the uteri were removed, dissected free of extraneous tissue, and the fluid contents were expelled before determination of wet weight in order to confirm estrogen deficiency associated with complete ovariectomy. Uterine weight was routinely reduced about 75% in response to ovariectomy. The uteri were then placed in 10% neutral buffered formalin to allow for subsequent histological analysis.

The right femurs are excised and scanned at the distal metaphysis proximal from the growth plate region by grey scale image analysis of digitalized X-ray generation on a Nicolet NXR-1200 real time X-ray imaging system. Additional image analysis was performed with NIH Image (1.45) software Relative density of bone was assayed over the lower end of the grey scale (therefore highest density range to demonstrate activity).

One of the major concepts put forth in the IDM of the use of Raloxifene and Bis-phosphonates for the treatment of osteoporosis was the concept of limiting the exposure of the patient to the potential side-effects of bis-phosphonates. We have done some further studies combinding other hormonally acting drugs with bis-phosphonates to see if Raloxifene has a unique profile when used in this modality. The data presented below demonstrate that raloxifene when used in combination with alendronate does have a different and more beneficial profile.

In Table 1, Alendronate (ALN) was combind with Provera (a synthetic Progestin) and tested in the 5-week ovex rat model of post-menopausal osteoporosis. As can be seen, at the two doses of Provera (1 and 10 mg/kg) and ALN at 0.1mg/kg, there was no protection from bone loss. The high dose of ALN (1mg/kg) had to be used to gain protection, even though at 10 mg/kg of provera had a protective effect by itself. This in contrast to RAL and ALN (93-11), where the low dose of RAL (0.1mg/kg) and ALN (0.1mg/kg) gave some protection from bone loss. At the high doses of either compound when given in combination, there seems to be little contribution by the Provera.

In Table 2, Alendronate was combined with ethynylestradiol (EE2, a synthetic estrogen), these results were similar to the raloxifene and alendronate combination, except that the total protection with Raloxifene and alendronate was superior. Again keeping in mind the concept of limiting the exposure to alendronate, EE₂ at both 30 and 100 ug/kg and alendronate at 0.1mg/kg could not achieve the complete protection of bone loss seen with the intact controls. The dose of Alendronate had to be increased to see that level of activity. In contast, in Table 3, virtually complete protection could be achieved with Raloxifene at 1 mg/kg and alendronate at 0.1 mg/kg.

In summary, each of the four agents tested could afford some level of protection against bone loss in this model when used alone. Alendronate and Provera demonstrated the least interactive effects. EE₂ and raloxifene plus alendronate did show interactive effects. However, raloxifene and alendronate in combination demonstrated the greatest protection from bone loss with the lowest exposure to the potentially undesirable side-effects of alendronate.

(It should be noted that when viewing the attached biological data @0.1mg/kg of alendronate and 0.1 mg/kg of raloxifene, this combination gave good anti-resorptive activity even though each compound separately was inactive. The molar ratio of the two compounds was 1:2 (raloxifene:alendronate) in this assay. It would seem very likely that a salt form with a 1:1 molar ratio, given at a slightly higher dose would be effective, whereas the two compounds if given separately might not.)

**TABLE 1**

| | | | |
|---|---|---|---|
| X-RAY-VOLTAGE/CONTRAST | | 47KV/4.5C | |
| | | | |

| GROUP | DOSE | RANGE | ±STD. ERR |
|---|---|---|---|
| | | 0-50 | |
| | | | |
| INTACT | | 58.81* | ±7.55 |
| OVEX CONTROL | CDX PO | 19.85 | ±6.27 |
| ALN | 0.1mg/kg PO CDX | 37.47 | ±7.08 |
| ALN | 1mg/kg PO CDX | 73.79* | ±7.73 |
| Provera | 1mg/kg PO CDX | 30.10 | ±5.61 |
| Provera | 10mgmg/kg PO CDX | 64.16* | ±6.78 |
| ALN + Provera | 0.1mg/kg + 1mg/kg PO CDX | 35.46 | ±4.85 |
| ALN + Provera | 0.1mg/kg + 10mg/kg PO CDX | 38.89 | ±9.67 |
| ALN + Provera | 1mg/kg + 10mg/kg PO CDX | 100.81* | ±4.43 |
| ALN + Provera | 1mg/kg + 10mg/kg PO CDX | 105.87* | ±2.92 |
| RAL | 1mg/kg PO CDX | 57.67* | ±10.25 |
| EE2 | 100µg/kg PO CDX | 51.29* | ±6.07 |
| | | | |
| *:P<=.05, TWO TAILED STUDENT T ON RAW DATA | | | |

**TABLE 2**

| | | | |
|---|---|---|---|
| | | | |
| X-RAY-VOLTAGE CONTRAST | | 47KV/4.5C | |
| | | | |

| GROUP | DOSE | RANGE | ±STD. ERR |
|---|---|---|---|
| | | 0-50 | |
| | | | |
| INTACT | | 84.40* | ±7.70 |
| OVEX CONTROL | CDX PO | 34.24 | ±7.53 |
| ALN | 0.1mg/kg PO CDX | 39.47 | ±2.39 |
| ALN | 1mg/kg PO CDX | 65.60* | ±3.71 |
| EE2 | 100µg/kg PO CDX | 61.09* | ±6.72 |
| EE2 | 30µg/kg PO CDX | 50.87 | ±7.89 |
| ALN + EE2 | 0.1mg/kg + 30µg/kg PO CDX | 57.82* | ±5.86 |
| ALN + EE2** | 0.1mg/kg + 100µg/kg PO CDX | 51.14 | ±11.09 |
| ALN + EE2 | 1mg/kg + 30µg/kg PO CDX | 86.93* | ±4.71 |
| ALN + EE2 | 1mg/kg + 100µg/kg PO CDX | 97.54* | ±3.37 |
| RAL | 1mg/kg PO CDX | 80.98* | ±5.25 |
| | | | |
| *:P<=.05, TWO TAILED STUDENT T ON RAW DATA | | | |
| **1/5 DEAD | | | |

**TABLE 3**

| | | | |
|---|---|---|---|
| | | | |
| | | | |
| X-RAY-VOLTAGE CONTRAST | | 47KV/4.5C | |
| | | | |

| GROUP | DOSE | RANGE | ±STD. ERR |
|---|---|---|---|
| | | 0-60 | |
| | | | |
| INTACT | | 71.72* | ±5.66 |
| OVEX CONTROL | CDX PO | 31.49 | ±7.27 |
| ALN | 0.1mg/kg PO CDX | 47.25 | ±6.15 |
| ALN | 1mg/kg PO CDX | 78.61* | ±7.26 |
| RAL | 0.1 mg/kg PO CDX | 36.76 | ±6.36 |
| RAL | 1mg/kg PO CDX | 40.37 | ±3.27 |
| RAL + ALN | 0.1mg/kg + 0.1mgkg PO CDX | 59.41* | ±9.12 |
| RAL + ALN | 0.1mg/kg + 1mg/kg PO CDX | 104.58* | ±7.97 |
| RAL + ALN | 1mg/kg + 0.1mg/kg PO CDX | 67.21* | ±6.77 |
| RAL + ALN | 1mg/kg + 1mg/kg PO CDX | 95.62* | ±4.02 |
| EE2 | 100µg/kg PO CDX | 62.88* | ±11.34 |
| | | | |
| *:P<=.05, TWO TAILED STUDENT T ON RAW DATA | | | |

## Claims

1. The use of a first compound which is a 2-phenyl-3-aroylbenzo[b]thiophene having the formula 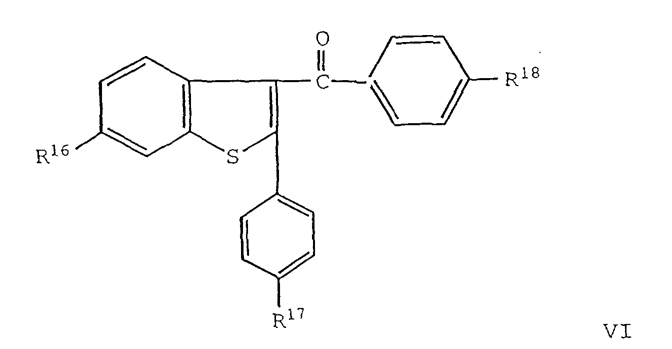 where
R¹⁶ is hydrogen, hydroxy or C₁-C₅ alkoxy C₁-C₇ alkanoyloxy, C₃-C₇ cycloalkanoyloxy, (C₁-C₆ alkoxy)-C₁-C₇ alkanoyloxy, substituted or unsubstituted aroyloxy, or substituted or unsubstituted aryloxycarbonyloxy;
R¹⁷ is hydrogen, hydroxy, C₁-C₅ alkoxy, adamantoyloxy, chloro, bromo, C₁-C₇ alkanoyloxy, C₃-C₇ cycloalkanoyloxy, (C₁-C₆ alkoxy)-C₁-C₇ alkanoyloxy, substituted or unsubstituted aroyloxy, or substituted or unsubstituted aryloxycarbonyloxy;
R¹⁸ is -O-CH₂-CH₂-X'-NR¹⁹R²⁰;
X' is a bond or -CH₂-, R¹⁹ and R²⁰ are independently C₁-C₄ alkyl or are taken together with the nitrogen atom to which they are bonded to constitute a pyrrolidinyl, piperidinyl, hexamethyleneiminyl, or morpholinyl ring; and pharmaceutically acceptable acid addition salts and solvates thereof in the preparation of a medicament in a synergistic combination with a second compound being a bisphosphonate, and pharmaceutically acceptable salts and solvates thereof, for inhibiting bone loss in a human.

2. The use of Claim 1 wherein said bisphosphonate is selected from alendronate, pamidronate, risedronate, cycloheptyl amino methyl idine bisphosphonate, and 3-pyrolidenyl-1-hydroxy propylidene bisphosphonate.

3. The use of any one of Claims 1 and 2, wherein said 2-phenyl-3-aroylbenzo[b]thiophene is [6-hydroxy-2-(4-hydroxyphenyl)benzo[b]thien-3-yl] [4-[2-(1-piperidinyl)ethoxy]phenyl]methanone and, pharmaceutically acceptable salts and solvates thereof.

4. The use of a bisphosphonate compound in the manufacture of a medicament in a synergistic combination with a 2-phenyl-3-aroylbenzo[b]thiophene compound as defined in claim 1, for inhibiting bone loss.

5. The use of any one of claims 1 to 4, wherein the 2-phenyl-3-aroylbenzo[b]thiophene compound is raloxifene.

6. The use of any one of claims 1 to 5, wherein the bisphosphonate compound is alendronate.

7. The use of a 2-phenyl-3-aroylbenzo[b]thiophene compound as defined in any one of claims 1 and 5 and a bisphosphonate compound as defined in any one of claims 1, 2 and 6, in the manufacture of a medicament for inhibiting bone loss.

8. A pharmaceutical formulation adapted for inhibiting bone loss in a human, comprising a synergistic combination of a first compound which is a 2-phenyl-3-aroylbenzo[b]thiophene compound as defined in any one of claims 1 and 5 and a second compound which is a bisphosphonate compound as defined in any one of claims 1, 2 and 6.

9. A salt formation product comprising a 2-phenyl-3-aroylbenzo[b]thiophene compound as defined in any one of claims 1 and 5 and a bisphosphonate compound as defined in any one of claims 1, 2 and 6.

10. A pharmaceutical formulation as claimed in claim 8, adapted for the separate co-administration of said first and second compounds.

11. A pharmaceutical formulation as claimed in claim 8, in the form of a simple mixture of said first and second compounds.

12. A pharmaceutical formulation as claimed in claim 8, in the form of a single molar salt formulation comprising said first and second compounds.

## Patentansprüche

1. Verwendung einer Verbindung, die ein 2-Phenyl-3-aroylbenzo[b]thiophen der folgenden Formel 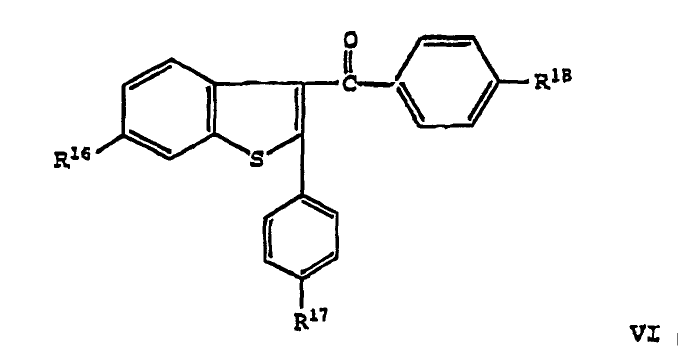 worin
R¹⁶ Wasserstoff, Hydroxy oder C₁-C₅-Alkoxy-C₁-C₇-alkanoyloxy, C₃-C₇-Cycloalkanoyloxy, (C₁-C₆-Alkoxy)-C₁-C₇-alkanoyloxy, substituiertes oder unsubstituiertes Aroyloxy oder substituiertes oder unsubstituiertes Aryloxycarbonyloxy ist,
R¹⁷ Wasserstoff, Hydroxy, C₁-C₅-Alkoxy, Adamantoyloxy, Chlor, Brom, C₁-C₇-Alkanoyloxy, C₃-C₇-Cycloalkanoyloxy, (C₁-C₆-Alkoxy)-C₁-C₇-alkanoyloxy, substituiertes oder unsubstituiertes Aroyloxy oder substituiertes oder unsubstituiertes Aryloxycarnoyloxy ist, und
R¹⁸ für -O-CH₂-CH₂-X'-NR¹⁹R²⁰ steht,
worin X' eine Bindung oder -CH₂- ist und R¹⁹ und R²⁰ unabhängig C₁-C₄-Alkyl sind oder zusammen mit dem Stickstoffatom, an das sie gebunden sind, einen Pyrrolidinyl-, Piperidinyl-, Hexamethyleniminyl oder Morpholinylring darstellen, oder
ein pharmazeutisch annehmbares Säureadditionssalz oder Solvat hiervon ist, zur Herstellung eines Arzneimittels in einer synergistischen Kombination mit einer zweiten Verbindung, die ein Bisphosphonat oder ein pharmazeutisch annehmbares Salz oder Solvat hiervon ist, zur Hemmung von Knochenverlust bei einem Menschen.

2. Verbindung nach Anspruch 1, worin das Bisphosphonat ausgewählt ist aus Alendronat, Pamidronat, Risedronat, Cycloheptylaminomethylidinbisphosphonat und 3-Pyrolidenyl-1-hydroxypropylidenbisphosphonat.

3. Verwendung nach einem der Ansprüche 1 und 2, worin das 2-Phenyl-3-aroylbenzo[b]thiophen [6-Hydroxy-2-(4-hydroxyphenyl)benzo[b]thien-3-yl][4-[2-(1-piperidinyl)ethoxy]phenyl]methanon oder ein pharmazeutisch annehmbares Salz oder Solvat hiervon ist.

4. Verwendung einer Bisphosphonatverbindung zur Herstellung eines Arzneimittels in einer synergistischen Kombination mit einer 2-Phenyl-3-aroylbenzo[b]thiophenverbindung gemäß Definition nach Anspruch 1 zur Hemmung von Knochenverlust.

5. Verwendung nach einem der Ansprüche 1 bis 4, worin die 2-Phenyl-3-aroylbenzo[b]thiophenverbindung Raloxifen ist.

6. Verwendung nach einem der Ansprüche 1 bis 5, worin die Bisphosphonatverbindung Alendronat ist.

7. Verwendung einer 2-Phenyl-3-aroylbenzo[b]thiophenverbindung gemäß Definition nach einem der Ansprüche 1 und 5 und einer Bisphosphonatverbindung gemäß Definition nach einem der Ansprüche 1, 2 und 6 zur Herstellung eines Arzneimittels zur Hemmung von Knochenverlust.

8. Pharmazeutische Formulierung, angepasst zur Hemmung von Knochenverlust bei einem Menschen, enthaltend eine synergistische Kombination aus einer ersten Verbindung, die eine 2-Phenyl-3-aroylbenzo[b]thiophenverbindung gemäß Definition nach einem der Ansprüche 1 und 5 ist, und aus einer zweiten Verbindung, die eine Bisphosphonatverbindung gemäß Definition nach einem der Ansprüche 1, 2 und 6 ist.

9. Salzformulierungsprodukt, enthaltend eine 2-Phenyl-3-aroylbenzo[b]thiophenverbindung gemäß Definition nach einem der Ansprüche 1 und 5 und eine Bisphosphonatverbindung gemäß Definition nach einem der Ansprüche 1, 2 und 6.

10. Pharmazeutische Formulierung nach Anspruch 8, angepasst zur getrennten Coverabreichung der ersten und der zweiten Verbindung.

11. Pharmazeutische Formulierung nach Anspruch 8 in Form eines einfachen Gemisches aus der ersten und der zweiten Verbindung.

12. Pharmazeutische Formulierung nach Anspruch 8 in Form einer einzelmolaren Salzformulierung, die die erste Verbindung und die zweite Verbindung enthält.

## Revendications

1. Utilisation d'un premier composé qui est un 2-phényl-3-aroylbenzo[b]thiophène ayant la formule 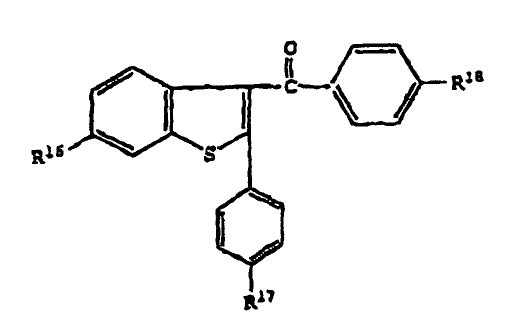 où
R¹⁶ est un atome d'hydrogène, un groupe hydroxy, alcoxy en C₁ à C₅, alcanoyloxy en C₁ à C₇, cycloalcanoyloxy en C₃ à C₇, (alcoxy en C₁ à C₆)-(alcanoyloxy en C₁ à C₇), aroyloxy substitué ou non substitué, ou aryloxycarbonyloxy substitué ou non substitué;
R¹⁷ est un atome d'hydrogène, un groupe hydroxy, alcoxy en C₁ à C₅, adamantoyloxy, chloro, bromo, alcanoyloxy en C₁ à C₇, cycloalcanoyloxy en C₃ à C₇, (alcoxy en C₁ à C₆)-(alcanoyloxy en C₁ à C₇), aroyloxy substitué ou non substitué ou aryloxycarbonyloxy substitué ou non substitué:
R¹⁸ est -O-CH₂-CH₂-X'-NR¹⁹R²⁰;
X' est une liaison ou -CH₂-, R¹⁹ et R²⁰ sont indépendamment un groupe alkyle en C₁ à C₄, ou sont pris conjointement avec l'atome d'azote auquel ils sont liés afin de constituer un cycle pyrrolidinyle, pipéridinyle, hexaméthylèneiminyle ou morpholinyle; et les solvates et sels d'addition d'acide de ceux-ci, acceptables sur le plan pharmaceutique dans la préparation d'un médicament dans une association synergique avec un deuxième composé qui est un bisphosphonate, et les sels et solvates de ceux-ci acceptables sur le plan pharmaceutique, en vue d'inhiber une perte de substance osseuse chez un humain.

2. Utilisation selon la revendication 1, dans laquelle ledit bisphosphonate est choisi parmi l'alendronate, le pamidronate, le risédronate, le bisphosphonate de cycloheptylaminométhylidine et le bisphonate de 3-pyrrolidényl-1-hydroxypropylidène.

3. Utilisation selon l'une quelconque des revendications 1 et 2, dans laquelle ledit 2-phényl-3-aroylbenzo[b]thiophène est la [6-hydroxy-2-(4-hydroxyphényl)benzo[b]thién-3-yl][4-(2-(1-pypéridinyl)éthoxy)phényl)-méthanone et les sels et solvates de celle-ci acceptables sur le plan pharmaceutique.

4. Utilisation d'un composé bisphosphonate dans la fabrication d'un médicament dans une association synergique avec un composé 2-phényl-3-aroylbenzo[b]thiophène tel que défini selon la revendication 1, en vue d'inhiber la perte de substance osseuse.

5. Utilisation selon l'une quelconque des revendications 1 à 4, dans laquelle le composé 2-phényl-3-aroylbenzo[b]thiophène est le raloxifène.

6. Utilisation selon l'une quelconque des revendications 1 à 5, dans laquelle le composé bisphosphonate est l'alendronate.

7. Utilisation d'un composé 2-phényl-3-aroylbenzo[b]thiophène tel que défini selon l'une quelconque des revendications 1 à 5 et un composé bisphosphonate tel que défini selon l'une quelconque des revendications 1,2 et 6, dans la fabrication d'un médicament pour inhiber la perte de substance osseuse.

8. Formulation pharmaceutique adaptée en vue d'inhiber la perte de substance osseuse chez un humain, comprenant une association synergique d'un premier composé qui est un composé 2-phényl-3-aroylbenzo[b]thiophène tel que défini selon l'une quelconque des revendications 1 et 5 et d'un deuxième composé qui est un composé bisphosphonate tel que défini selon l'une quelconque des revendications 1, 2 et 6.

9. Produit de formation d'un sel, comprenant un composé 2-phényl-3-aroylbenzo[b]thiophène tel que défini selon l'une quelconque des revendications 1 et 5 et un composé bisphosphonate tel que défini selon l'une quelconque des revendications 1, 2 et 6.

10. Formulation pharmaceutique selon la revendication 8, adaptée à la co-administration séparée desdits premier et deuxième composés.

11. Formulation pharmaceutique selon la revendication 8, sous la forme d'un simple mélange desdits premier et deuxième composés.

12. Formulation pharmaceutique selon la revendication 8, sous la forme d'une simple formulation de sel molaire comprenant lesdits premier et deuxième composés.
